# EUROPEAN PATENT SPECIFICATION

(11) **EP 1 896 044 B1**
(45) Date of publication and mention of the grant of the patent: **23.03.2011**
(21) Application number: 05765896.5
(22) Date of filing: 27.06.2005
(51) Int. Cl.: C12N 5/071

(54) **BONE FORMATION COMPOSITION COMPOSED OF MIXTURE OF OSTEOBLAST AND BIO-MATRIX AND ITS MANUFACTURING METHOD**
KNOCHENBILDUNGSZUSAMMENSETZUNG AUS OSTEOBLASTENGEMISCH UND BIOMATRIX UND HERSTELLUNGSVERFAHREN DAFÜR
COMPOSITION DE FORMATION OSSEUSE CONSTITUEE D'UN MELANGE D'OSTEOBLASTES ET DE BIOMATRICE, ET SON PROCEDE DE FABRICATION

(30) Priority: 13.06.2005 KR 20050050447
(43) Date of publication of application: 12.03.2008
(73) Proprietor: Sewon Cellontech Co., Ltd., Youngdeungpo-Gu, Seoul 150-712 (KR)
(72) Inventor: JANG, Jae-Deog, Nowon-gu, Seoul 139-784 (KR); PARK, Hyun-Shin, Yangcheon-gu, Seoul 158-774 (KR); CHANG, Cheong-Ho, Seocho-gu, Seoul 137-040 (KR); JUNG, Soo-Jin, 301-1103 Sinmyoung APT, Guri-si, Gyeonggi-do 471-020 (KR); LEE, Sae-Bom, Gwangjin-gu, Seoul 143-769 (KR); KO, Chang-Kwon, Nwon-gu, Seoul 139-921 (KR)
(74) Representative: Merkle, Gebhard
(86) International application number: PCT/KR2005/002006
(87) International publication number: WO 2006/135123

(56) References cited:
- EP-A- 1 535 633
- WO-A-01/85225
- WO-A-03/007873
- WO-A-2004/110512
- DE-A1- 19 949 290
- KR-A- 2003 081 821
- US-A- 5 942 496
- ENDRES G F ET AL: "Equilibria in the fibrinogen-fibrin conversion - IX. Effects of calcium ions on the reversible polymerization of fibrin monomer" ARCHIVES OF BIOCHEMISTRY AND BIOPHYSICS, ACADEMIC PRESS, US, vol. 153, no. 1, 1 November 1972 (1972-11-01), pages 266-278, XP024805130 ISSN: 0003-9861 [retrieved on 1972-11-01]
- APAP-BOLOGNA A ET AL: "The influence of calcium ions on fibrinogen conformation" BIOCHIMICA ET BIOPHYSICA ACTA - PROTEIN STRUCTURE AND MOLECULAR ENZYMOLOGIE, ELSEVIER SCIENCE BV, AMSTERDAM, NL, vol. 995, no. 1, 16 March 1989 (1989-03-16), pages 70-74, XP023579729 ISSN: 0167-4838 [retrieved on 1989-03-16]
- KNESER U: "FIBRIN GEL-IMMOBILIZED PRIMARY OSTEOBLASTS IN CALCIUM PHOSPHATE BONE CEMENT: IN VIVO EVALUATION WITH REGARD TO APPLICATION AS INJECTABLE BIOLOGICAL BONE SUBSTITUTE" CELLS TISSUES ORGANS, KARGER, BASEL, CH, vol. 179, no. 4, 1 January 2005 (2005-01-01), pages 158-169, XP008074203 ISSN: 1422-6405
- XIAOHUA LIU ET AL: "Polymeric Scaffolds for Bone Tissue Engineering" ANNALS OF BIOMEDICAL ENGINEERING, KLUWER ACADEMIC PUBLISHERS-PLENUM PUBLISHERS, NE, vol. 32, no. 3, 1 March 2004 (2004-03-01), pages 477-486, XP019272806 ISSN: 1573-9686
- HSU F.Y. ET AL.: 'Microspheres of hydroxyapatite/reconstituted collagen as supports for osteoblast cell growth' BIOMATERIALS vol. 20, no. 20, 1999, pages 1931 - 1936, XP002388329
- DU C; ET AL: "THREE-DIMENSIONAL NANO-HAP/COLLAGEN MATRIX LOADING WITH OSTEOGENIC CELLS IN ORGAN CULTURE" JOURNAL OF BIOMEDICAL MATERIALS RESEARCH, vol. 44, 1999, pages 407-415,
- DU C; ET AL: "FORMATION OF CALCIUM PHOSPHATE/COLLAGEN COMPOSITES THROUGH MINERALIZATION OF COLLAGEN MATRIX" JOURNAL OF BIOMEDICAL MATERIALS RESEARCH, vol. 50, 2000, pages 518-527,
- BELL R; BEIRNE O R: "Effect of hydroxylapatite, tricalcium phosphate, and collagen on the healing of defects in the rat mandible" JOURNAL OF ORAL AND MAXILLOFACIAL SURGERY, vol. 46, 1988, pages 589-594,
- LIAO S S; CUI F Z; ZHANG W; FENG Q L: "Hierarchically biomimetic bone scaffold materials: nano-HA/collagen/PLA composite" JOURNAL OF BIOMEDICAL MATERIALS RESEARCH, vol. 69, 2004, pages 158-165,

## Description

### [Technical Field]

The present invention relates to a composition for bone formation using a mixture of osteoblasts and bio-matrix, and a method for preparing the same. More specifically, the present invention relates to a composition for bone formation using a mixture of osteoblasts and a bio-matrix, which can be transplanted for bone formation upon treating bone defects or into a region in need thereof, and a method for preparing the same.

### [Background Art]

According to the reports issued by the World Health Organization (WHO), over half of the aged over the age of 65 suffer from chronic bone diseases, and the incidence of bone fracture related to osteoporosis has doubled over the past decade. These figures correspond to approximately 40% of women 50 and older.

In the USA, roughly 5.60 million people suffer bone fractures a year and 3.10 million of them have surgical operations. According to a statistical report of the Medical Data International (MDI) in 1995, about 426,000 cases of bone graft surgeries were conducted. Costs for bone grafting are estimated to be approximately 800 million US dollars a year throughout the world. For just one year of 1995, bone grafting including 58% of autografting, 34% of allografting and 8% of synthetic material transplantation was conducted.

Generally, a simple (closed) bone fracture may be sufficiently healed by casting the bone fracture for several weeks, but a severe bone fracture or bone defect requires bone grafting.

However, such autografting disadvantageously may cause strong pain in a bone-harvested region and require a long period of time for recovery after surgical operation for bone grafting, and has suffered from a great deal of difficulty to secure donor parts for providing bone for bone transplantation.

Meanwhile, allografting also suffers from fatal disadvantages such as weakening of bone strength during a sterilization process, occurrence of graft rejection, and possibility of transmission with contagious diseases such as hepatitis and AIDS.

As other available methods for bone grafting, metals on which biologically active or biologically non-active ceramic materials are coated are widely used as supporting materials in the orthopedic surgery, but there is a great deal of difficulty to use metals as bone transplants due to problems such as corrosion of metals, wear of the ceramic-metal surface, and severe formation of fibrose tissues at surfaces of the bones and transplants.

For bone-formation factors, although a great deal of studies have been made on various factors after Urist and Mclean (1952) have issued a publication of effects of bone formation proteins on bone-formation, the production process thereof is very complicated and expensive, and has low efficiency, thus resulting in a low yield and ultimately limiting application thereof to a practical clinical use.

Meanwhile, bone marrow injection is a technique based on an assertion, proposed by Huggins (1931), Friedenstein (1973), and Ashton (1980), wherein osteoprogenitor cells from bone marrow induce and facilitate bone formation. Bone marrow injection is generally carried out alone for healing bone fractures, but is also carried out in combination with bone grafting. Unlike other bone grafting techniques, this bone marrow injection does not involve surgical skin incision for donor parts and thus is significantly advantageous due to no problems associated with securing of donor parts and no complications or adverse side effects.

Thereafter, even though some excellent results are published through a great deal of clinical application cases, bone marrow injection is disadvantageous due to its unsound theoretical basis, by the reasons that considerable numbers of results obtained are not consistent therebetween, the amount of bone marrow collectable from one site is limited and further, there is a significantly limited number of osteoprogenitor cells contained in bone marrow.

As such, a novel bone formation and transplantation method by culturing and amplifying osteoprogenitor cells into sufficient numbers of osteoblast cells, mixing the cultured cells with a bio-matrix and injecting the resulting mixture into a bone-formation region to be sought is a highly effective method, implies significant advantages and beneficial effects, as compared to conventional autografting or allografting and bone marrow injection, and thus is a tissue engineering field receiving a great deal of attention in bone regeneration therapies.

### [Disclosure]

### [Technical Problem]

Therefore, the present invention has been made in view of the problems associated with implants and conventional bone grafting techniques as discussed above, and it is an object of the present invention to provide a composition for bone formation using a mixture of osteoblasts and bio-matrix, and a method for preparing the same, resulting in no clinical graft rejection via injection of osteoblasts and bio-matrix mixture for bone formation into a site where bone formation is sought, and being capable of achieving effective and rapid bone formation via injection of a composition which was shaped to a certain extent, so as to alleviate problems associated with probability of bone tissue formation in unwanted regions resulting from escape of injected osteoblasts from the desired site for bone formation and then propagation thereof to other sites via the blood stream, which may be caused by injection of an osteoblast suspension.

### [Technical Solution]

In accordance with an aspect of the present invention, the above and other objects can be accomplished by the provision of a method for preparing a composition for bone formation, comprising:
isolating osteoblasts and their precursor cells from a bone tissue and culturing/proliferating the isolated osteoblasts and their precursor cells in DMEM (Dulbecco's Modified Eagle's Medium) or α-MEM (Minimum Essential Medium, Alpha Modification) to prepare an osteoblast suspension; and
mixing the resulting osteoblast suspension with a bio-matrix to prepare an osteoblast therapeutic agent.

Herein, the preparation step of the therapeutic agent further includes mixing the osteoblast mixed solution, in which the bio-matrix was mixed in the osteoblast suspension, with a coagulant.

Herein, the mixing step includes mixing the osteoblast mixed solution with 10 to 100 IU/mL of thrombin as a coagulant; and mixing the resulting mixed solution containing thrombin mixed therein with 20 to 100 mg/mL of fibrinogen as the coagulant.

Herein, the bio-matrix is collagen, hydroxyapatite or a mixture thereof.

Herein, collagen may be added in an amount of 67 µg/mL to 20 mg/mL to the osteoblast suspension, and hydroxyapatite may be added in an amount of 30 µg/mL to 3.4 mg/mL to the osteoblast suspension.

Collagen is neutralized to a neutral pH by addition of a neutralization solution prior to mixing with the osteoblast suspension.

Prior to adding to the mixed solution of the osteoblast and bio-matrix, lyophilized thrombin as thrombin is dissolved in liquid DMEM or α-MEM to which phosphate (PO₄³⁻) ions were added as a bone mineral component, and is added to be double an amount of thrombin contained in the culture medium to which (PO₄³⁻) ions were added. Then, prior to adding to the resulting osteoblast mixed solution, lyophilized fibrinogen as fibrinogen is dissolved in liquid DMEM or α-MEM to which calcium (Ca²⁺) ions were added as a bone mineral component, and is added to be double an amount of fibrinogen contained in the culture medium to which calcium (Ca²⁺) ions were added.

In accordance with another aspect of the present invention, there is provided a composition for bone formation prepared by the above method for preparing a composition for bone formation using a mixture of an osteoblast and a bio-matrix.

### [Advantageous Effects]

In accordance with a composition for bone formation using a mixture of an osteoblast and a bio-matrix, and a method for preparing the same, having a construction as described above, it is possible to achieve bone formation, resulting in no clinical graft rejection via injection of an osteoblast and bio-matrix mixture for bone formation into a site where bone formation is sought, and being capable of achieving effective and rapid bone formation via injection of a composition which was shaped to a certain extent, so as to alleviate problems associated with probability of bone tissue formation in unwanted regions resulting from escape of injected osteoblasts from the desired site for bone formation and then propagation thereof to other sites via the blood stream, which are caused by injection of an osteoblast suspension.

### [Description of the Drawings]

The above and other objects, features and other advantages of the present invention will be more clearly understood from the following detailed description taken in conjunction with the accompanying drawings, in which:
Fig. 1 is a bar graph showing a composition ratio of bone;
Fig. 2 is a process flow chart illustrating a method for preparing a composition for bone formation using a mixture of an osteoblast and a bio-matrix in accordance with the present invention;
Fig. 3 is a photograph illustrating subcutaneous injection of an osteoblast therapeutic agent, which is a mixture of an osteoblast and a bio-matrix, into an immunodeficient mouse;
Fig. 4 is a photograph of an immunodeficient mouse taken 4 weeks after transplantation of an osteoblast therapeutic agent, which is a mixture of an osteoblast and a bio-matrix;
Fig. 5 is a photograph of a transplant taken 4 weeks after transplantation of an osteoblast therapeutic agent, which is a mixture of an osteoblast and a bio-matrix, into an immunodeficient mouse;
Fig. 6 is an autoradiograph of an immunodeficient mouse taken 4 weeks after transplantation of an osteoblast therapeutic agent, which is a mixture of an osteoblast and a bio-matrix;
Fig. 7 is a photograph of a tissue section stained with Hematoxylin-Eosin capable of confirming bone formation, taken 8 weeks after transplantation of an osteoblast therapeutic agent, which is a mixture of an osteoblast and a bio-matrix, into an immunodeficient mouse; and
Fig. 8 is a photograph of a tissue section stained with Masson's Trichrome, taken 8 weeks after transplantation of an osteoblast therapeutic agent, which is a mixture of an osteoblast and a bio-matrix, into an immunodeficient mouse.

### [Best Mode]

Hereinafter, a method for preparing a composition for bone formation using a mixture of an osteoblast and a bio-matrix in accordance with the present invention will be described in more detail with reference to the accompanying drawings.

In order to overcome disadvantages exhibited by conventional therapies for treating bone-related disorders and diseases, such as implants and bone grafting, a technique of transplanting autologous osteoblasts having no adverse side effects on an affected part, via culturing of osteoblasts, as an ultimate treatment method, has been developed, in conjunction with improvement in conventional therapies.

However, for achieving faster bone formation, it is necessary to mix osteoblasts with a matrix through which more diverse lesions can be treated, and thereby it is possible to treat more bone diseases that do not achieve therapeutic benefits by injection of a liquid osteoblast suspension consisting of osteoblasts only.

It can be said that the osteoblast therapeutic agent, which is a mixture of an osteoblast and a bio-matrix, is a more advanced version of an injection method of a liquid osteoblast suspension which is based on cell therapy. The conventional injection method of a liquid osteoblast suspension, which involves transplanting osteoblasts alone, can only treat limited types of bone defects and also suffers from problems associated with probability of bone tissue formation in unwanted regions resulting from escape of injected osteoblasts from desired bone-forming sites and then propagation thereof to other sites via the blood stream. In contrast, the osteoblast therapeutic agent, which is a mixture of an osteoblast and a bio-matrix, in accordance with the present invention, is made up of the osteoblast and bio-matrix mixture and therefore, can also more rapidly and effectively treat a broader range of bone defects and severe bone diseases.

Bones are composed of bone cells and large quantities of bone matrices present between cells. Most parts of bone matrices are comprised of organic components (35%) made up of collagen fibers and inorganic components (65%) made up of calcium and phosphate.

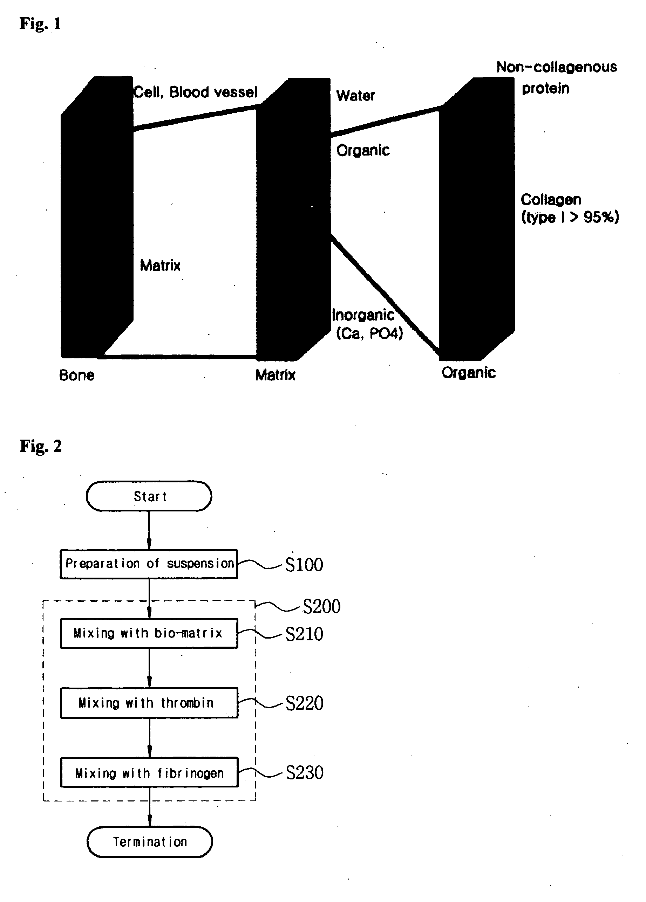

Bones, as shown in Fig. 1, are biosynthetic substances and are composed of minerals, collagen, moisture, non-collagenous proteins, lipids, vascular components and cells, in order of the highest composition ratio to the lowest.

The inorganic matter taking the largest portions of bone composition is an analogue of hydroxyapatite (HA) that is a geological mineral.

Bone apatite is generally deficient in calcium and hydroxyl groups, which are replaced by numerous impurities including carbonate and other magnesium, potassium, boron, phosphate and citrate, the most abundant of which is carbonate.

A content of carbonate in the bone minerals increases with maturation of bones. Carbonate replaces hydroxyl or phosphate groups, or may be adsorbed on the surface of bone apatite.

The second most abundant constituent of bones is collagen, mainly type I collagen. Collagen imparts elasticity and flexibility to bones and offers direction for matrix constitution.

According to the embodiment of the present invention, osteoblasts are mixed with the bio-matrix and the resulting mixture should be injected into regions suffering from bone diseases, and collagen and hydroxyapatite, which are bone components, are mixed and injected into lesions. Therefore, it is possible to secure physical properties of bones in advance and thereby realize faster bone formation and adequately mineralized bones.

In mixing the osteoblasts, a main component of the bone matrix, with the bio-matrix, one of the most crucial factors is stability. In order to ensure sufficient numbers of the cells within the matrix, the survival rate of cells should be enhanced. In addition, it is also important to increase the engrafting rate of the cells after performing transplantation thereof.

Fig. 2 is a process flow chart illustrating a method for preparing a composition for bone formation using a mixture of an osteoblast and a bio-matrix in accordance with the present invention.

Referring to Fig. 2, the method for preparing a composition for bone formation in accordance with the present invention comprises the steps of isolating osteoblasts from a bone tissue and culturing/proliferating the isolated osteoblasts in DMEM (Dulbecco's Modified Eagle's Medium) or α-MEM (Minimum Essential Medium, Alpha Modification) to prepare an osteoblast suspension (S100); and mixing the resulting osteoblast suspension with a bio-matrix to prepare an osteoblast therapeutic agent (S200).

Herein, the number of osteoblasts in the osteoblast suspension may significantly vary depending upon sizes and locations of lesions of interest. Typically, the number of osteoblasts is preferably in a range of 10⁶ to 1.2 x 10⁷ cells, although a higher number of cells may be used.

Herein, the preparation step (S200) of the therapeutic agent further includes mixing the osteoblast/bio-matrix mixed solution, in which the bio-matrix was mixed in the osteoblast suspension, with a coagulant.

Herein, the mixing step includes mixing the osteoblast/bio-matrix mixed solution with 10 to 100 IU/mL of thrombin as the coagulant (S220), and mixing the resulting mixed solution containing thrombin mixed therein with 20 to 100 mg/mL of fibrinogen as the coagulant (S230).

As the bio-matrix, collagen, hydroxyapatite or a mixture thereof may be used. When the mixture of collagen and hydroxyapatite is used as the bio-matrix, 67 µg/mL to 20 mg/mL of collagen is mixed with 30 µg/mL to 3.4 mg/mL of hydroxyapatite.

Meanwhile, a minimal concentration of collagen was an optimal culture concentration inducing cell differentiation and proliferation when collagen was used as a coating material for coating a culture vessel in culturing osteoblasts. When the collagen concentration applied at this time was applied as the matrix component, it could be confirmed that in injecting the composition in which osteoblasts and bio-matrix were mixed with each other, the collagen components, which were injected before osteoblasts expressed collagen, form a basic matrix network, resulting in cell differentiation and rapid mineralization which consequently leads to rapid bone formation with a minimal amount of collagen. Meanwhile, where collagen was used as a sole matrix, the collagen concentration equal to or higher than 3 mg/mL (0.3%) leads to gelation under predetermined conditions, thereby forming a matrix. In contrast, a maximal concentration of 20 mg/mL of collagen results in a sharp decrease of fluidity thereof and thus osteoblasts are not homogeneously dispersed in the collagen matrix and it is thus difficult to apply the collagen as a cell matrix.

Further, hydroxyapatite should be added in a concentration such that hydroxyapatite promotes mineralization in a matrix mixture. Where an excess amount of hydroxyapatite is contained, this may inhibit cell differentiation due to spatial restriction which occurs due to occupation of hydroxyapatite in the cell matrix mixture. Therefore, in the present invention, hydroxyapatite was applied in a minimal concentration at which hydroxyapatite can serve as a nucleus for mineralization in the matrix component, and in an optimal concentration to promote mineralization.

On the other hand, collagen is neutralized to a neutral pH by addition of a neutralization solution prior to mixing with the osteoblast suspension.

In addition, prior to adding to the mixed solution of the osteoblasts and bio-matrix, lyophilized thrombin as thrombin is dissolved in liquid DMEM or α-MEM to which phosphate (PO₄³⁻) ions were added, and is added to be double an amount of thrombin contained in the culture medium to which (PO₄³⁻) ions were added. Then, prior to adding to the resulting osteoblast mixed solution, lyophilized fibrinogen as fibrinogen is dissolved in liquid DMEM or α-MEM to which calcium (Ca²⁺) ions were added, and is added to be double an amount of fibrinogen contained in the culture medium to which calcium (Ca²⁺) ions were added. Herein, phosphate ions and calcium ions exhibit no cytotoxic effects on osteoblasts and supplement bone mineral components.

Thrombin as the coagulant is mixed in an amount of 10 to 100 IU/mL in the mixed solution, and fibrinogen as the coagulant is mixed in an amount of 20 to 100 mg/mL in the resulting mixture containing thrombin mixed therein.

Meanwhile, a concentration of thrombin determines a polymerization time of fibrin. Therefore, the polymerization time of fibrin can be reduced within a range of 4 hours to 3 sec, depending upon the concentration of thrombin. Thrombin was applied to the concentration such that the composition of the present invention is shaped to prevent a mixed composition of osteoblasts and bio-matrix from leaking from lesion sites of interest when injected into a site for bone formation, injection of the composition into the affected site leads to rapid polymerization, and an optimal fibrin pore matrix for bone formation by osteoblasts is formed.

### [Mode for Invention]

### EXAMPLES

Hereinafter, effects of a composition for bone formation using a mixture of an osteoblast and a bio-matrix in accordance with an example of the present invention will be described in more detail with reference to the accompanying drawings.

### Example 1

Firstly, osteoblasts and their precursor cells were isolated from the corresponding tissues, and cultured and proliferated in DMEM or α-MEM for 4 weeks, thereby preparing an osteoblast suspension composed of DMEM or α-MEM. As a bio-matrix to be mixed therein, collagen was prepared.

Then, the resulting osteoblast suspension was mixed with collagen, to thereby prepare a total 1 mL of a composition for bone formation.

### Example 2

Osteoblasts and their precursor cells were isolated from the corresponding tissues, and cultured and proliferated in DMEM or α-MEM for 4 weeks, thereby preparing an osteoblast suspension composed of DMEM or α-MEM. As bio-matrices, collagen and hydroxyapatite were prepared. A 1/10 volume of hydroxyapatite was added to the osteoblast suspension and mixed well. As the bio-matrix to be mixed therein, 2/5 volumes of collagen were prepared.

The osteoblast suspension was mixed with collagen to prepare a total 1 mL of a composition for bone formation.

### Example 3

Osteoblasts and their precursor cells were isolated from the corresponding tissues, and cultured and proliferated in DMEM or α-MEM for 4 weeks, thereby preparing an osteoblast suspension composed of DMEM or α-MEM. As bio-matrices, collagen and hydroxyapatite were prepared. 2/5 volumes of collagen and a 1/10 volume of hydroxyapatite were added to the osteoblast suspension and mixed well, thereby forming an osteoblast therapeutic agent mixed with bio-matrices.

After preparing a medical-grade fibrin glue set at room temperature, fibrinogen was dissolved by adding a proper quantity of liquid DMEM or α-MEM to a vial containing lyophilized fibrinogen. In addition, thrombin was also dissolved by adding a proper quantity of liquid DMEM or α-MEM to a vial containing the lyophilized thrombin.

Then, a 1/10 volume of the dissolved thrombin was added to the liquid osteoblast suspension in which bio-matrices were mixed, and the resulting mixture was mixed well.

The osteoblast suspension mixed with thrombin and bio-matrices was mixed with an equal amount of dissolved fibrinogen to prepare a total 1 mL of a composition for bone formation.

### Example 4

Osteoblasts and their precursor cells were isolated from the corresponding tissues, and cultured and proliferated in DMEM or α-MEM for 4 weeks, thereby preparing an osteoblast suspension composed of DMEM or α-MEM. As bio-matrices, collagen and hydroxyapatite were prepared. 2/5 volumes of collagen and a 1 /10 volume of hydroxyapatite were added to the osteoblast suspension and mixed well, thereby forming an osteoblast therapeutic agent mixed with the bio-matrices.

After preparing a medical-grade fibrin glue set at room temperature, fibrinogen was dissolved by adding a proper quantity of liquid DMEM or α-MEM to a vial containing lyophilized fibrinogen. In addition, thrombin was also dissolved by adding a proper quantity of liquid DMEM or α-MEM to a vial containing the lyophilized thrombin.

At this time, the liquid DMEM or α-MEM was used to which phosphate (PO₄³⁻) ions were added to a concentration of 2 mg/mL. In addition, a proper quantity of liquid DMEM or α-MEM was added to a vial containing the lyophilized thrombin, thereby dissolving the thrombin. At this time, the liquid DMEM or α-MEM was used to which calcium (Ca²⁺) ions were added to a concentration of 4 mg/mL. Amounts of phosphate ions (PO₄³⁻) and calcium ions (Ca²⁺) used herein are optimal amounts which do not exhibit cytotoxic effects on osteoblasts and serve as additives for the culture solution to thereby supplement bone mineral components.

Then, the dissolved thrombin was added in a 1/10 volume to the liquid osteoblast suspension mixed with bio-matrices and mixed well.

The osteoblast suspension containing thrombin and bio-matrices mixed therein was mixed with an equal amount of dissolved fibrinogen to prepare a total 1 mL of a composition for bone formation.

### Results

15 immunodeficient mice were divided into 6 groups (triple trial). Then, an osteoblast therapeutic agent, which is a mixture of an osteoblast and a bio-matrix, was prepared and injected into the scapula of nude mice via subcutaneous injection. 1 mL of the osteoblast therapeutic agent was injected into the hypoderm of each immunodeficient mouse. The results were confirmed 4 weeks and 8 weeks after injection.

**Table 1**

| | Group 1 | Group 2 | Group 3 |
|---|---|---|---|
| Cell number (cells) | 1×10⁶ cells | 6×10⁶ cells | 1.2×10⁷ cells |
| Injection volume (cc) | 1 mL | 1 mL | 1 mL |

Table 1 shows respective osteoblast therapeutic agents having the same bio-matrix composition but different numbers of osteoblasts, which are mixtures of osteoblasts and bio-matrices, transplanted into immunodeficient mice.

**Table 2**

| | Group 1 | Group 2 | Group 3 |
|---|---|---|---|
| Size volume (cc) | 0.26 | 0.24 | 0.25 |
| Bone formation | Good | Good | Good |

Here, a bone formation grade is rated in order of excellent/good/fair/poor.

8 weeks after transplanting the osteoblast therapeutic agents as given in Table 1, the results for bone formation thus obtained are shown in Table 2.

**Table 3**

| | Group 4 | Group 5 | Group 6 |
|---|---|---|---|
| Cell number (cells) | 6×10⁶ cells | 6×10⁶ cells | 6×10⁶ cells |
| Collagen vol. | 1/5 | 2/5 | 1 |
| Injection volume (cc) | 1 mL | 1 mL | 1 mL |

Table 3 shows respective osteoblast therapeutic agents having different amounts of bio-matrices added, which are mixtures of osteoblasts and bio-matrices, transplanted into immunodeficient mice.

**Table 4**

| | Group 4 | Group 5 | Group 6 |
|---|---|---|---|
| Size volume(cc) | 0.24 | 0.52 | 0.50 |
| Bone formation | Good | Excellent | Excellent |

Here, a bone formation grade is rated in order of excellent/good/fair/poor.

8 weeks after transplanting the osteoblast therapeutic agents as given in Table 3, the results for bone formation thus obtained are shown in Table 4.

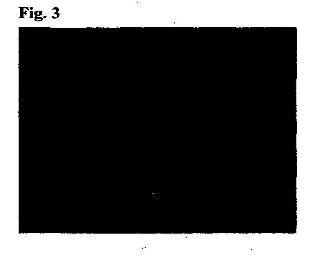

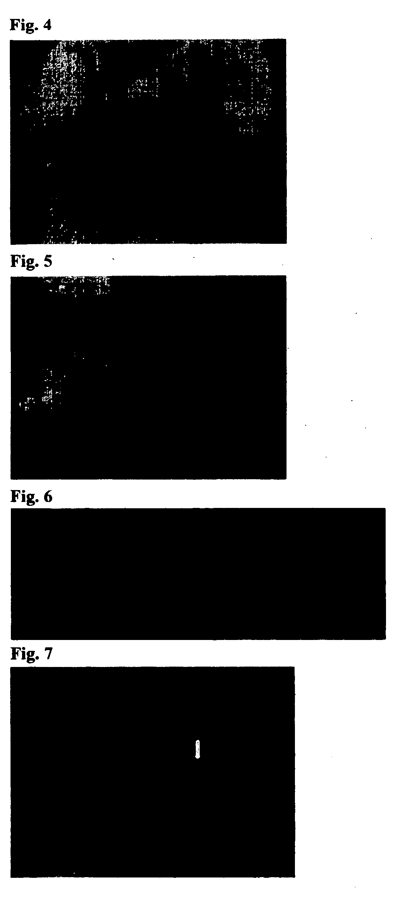

Fig. 3 is a photograph illustrating subcutaneous injection of an osteoblast therapeutic agent, which is a mixture of an osteoblast and a bio-matrix, into an immunodeficient mouse. Fig. 4 is a photograph of bone formation (observed as a bulged portion circumscribed by a dotted line) in an immunodeficient mouse taken 4 weeks after transplantation of an osteoblast therapeutic agent, which is a mixture of an osteoblast and a bio-matrix.

Fig. 5 is a photograph of a bone-formation transplant taken 4 weeks after transplantation of an osteoblast therapeutic agent, which is a mixture of an osteoblast and a bio-matrix, into an immunodeficient mouse, thus confirming that formation of blood vessels was induced around the transplant. Fig. 6 is an autoradiograph of an immunodeficient mouse taken 4 weeks after transplantation of an osteoblast therapeutic agent, which is a mixture of an osteoblast and a bio-matrix and it was observed that a new bone was formed on the backbone, as circumscribed by a dotted-line circle.

Fig. 7 is a photograph of a tissue section stained with Hematoxylin-Eosin capable of confirming bone formation, taken 8 weeks after transplantation of an osteoblast therapeutic agent, which is a mixture of an osteoblast and a bio-matrix, into an immunodeficient mouse. As can be seen, osteoid (represented by a block arrow) is observed and osteoblasts distributed within Lacuna (represented by a white arrow) can also be confirmed.

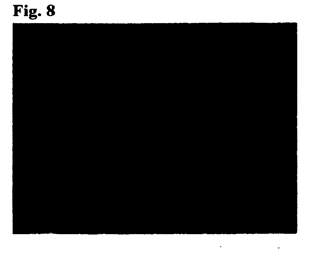

Fig. 8 is a photograph of a tissue section stained with Masson's Trichrome, taken 8 weeks after transplantation of an osteoblast therapeutic agent, which is a mixture of an osteoblast and a bio-matrix, into an immunodeficient mouse, and it can be confirmed that bone formation occurred in conjunction with collagen along predetermined patterns, thus showing penetration of blood vessels between matrices.

Thus, in accordance with the present invention, there is provided a bone formation method, resulting in no clinical graft rejection via injection of an osteoblast and bio-matrix mixture into a site where bone formation is sought, and being capable of achieving effective and rapid bone formation via injection of a composition which was shaped to a certain extent.

### [Industrial Applicability]

In accordance with the present invention, a composition for bone formation using a mixture of an osteoblast and a bio-matrix, and a method for preparing the same, having a construction as described above, enable realization of bone formation, resulting in no clinical graft rejection via injection of an osteoblast and bio-matrix mixture for bone formation into a site where bone formation is sought, and being capable of achieving effective and rapid bone formation via injection of a composition which was shaped to a certain extent, so as to alleviate problems associated with probability of bone tissue formation in unwanted regions resulting from escape of injected osteoblasts from the targeted site for bone formation and then propagation thereof to other sites via the blood stream, which are caused by injection of an osteoblast suspension.

Although the preferred embodiments of the present invention have been disclosed for illustrative purposes, those skilled in the art will appreciate that various modifications, additions and substitutions are possible.

## Claims

1. A composition for bone formation prepared by the method for preparing a composition for bone formation using a mixture of an osteoblast and a bio-matrix, comprising:
culturing/proliferating isolated osteoblasts and their precursor cells in alpha-MEM (Minimum Essential Medium, from a bone tissue Alpha Modification) to prepare an osteoblast suspension; and
mixing the resulting osteoblast suspension with a bio-matrix to prepare an osteoblast therapeutic agent,
wherein the bio-matrix is a mixture of collagen and hydroxyapatite,
wherein collagen is added in an amount of 67 µg/mL to 20 mg/mL to the osteoblast suspension, and hydroxyapatite is added in an amount of 30 µg/mL to 3.4 mg/mL to the osteoblast suspension.

2. The composition for bone formation according to claim 1, wherein the preparation step of the therapeutic agent further includes mixing the osteoblast mixed solution, in which the bio-matrix was mixed in the osteoblast suspension, with a coagulant.

3. The composition for bone formation according to claim 2, wherein the mixing step includes:
mixing the osteoblast mixed solution with 10 to 100 IU/mL of thrombin as the coagulant; and
mixing the resulting mixed solution containing thrombin mixed therein with 20 to 100 mg/mL of fibrinogen as the coagulant.

4. The composition for bone formation according to claim 1, wherein collagen is neutralized to a neutral pH by addition of a neutralization solution prior to mixing with the osteoblast suspension.

5. The composition for bone formation according to claim 3, wherein
prior to adding to the mixture of the osteoblast and bio-matrix, lyophilized thrombin as thrombin is dissolved in liquid alpha-MEM to which phosphate (PO₄³⁻) ions were added as a bone mineral component, and is added to be double an amount of thrombin contained in the culture medium to which phosphate (PO₄³⁻) ions were added; and
prior to adding to the resulting osteoblast mixed solution, lyophilized fibrinogen as fibrinogen is dissolved in liquid alpha-MEM to which calcium (Ca²⁺) ions were added as a bone mineral component , and is added to be double an amount of fibrinogen contained in the culture medium to which calcium (Ca²⁺) ions were added.

## Patentansprüche

1. Zusammensetzung zur Knochenbildung, hergestellt durch das Verfahren für die Herstellung einer Zusammensetzung zur Knochenbildung unter Verwendung eines Gemisches aus Osteoblasten und einer Biomatrix, welches umfasst:
das Züchten/Vermehren von Osteoblasten und deren Vorläuferzellen, welche aus einem Knochengewebe isoliert wurden, in alpha-MEM (Alpha-Modifikation des essentiellen Minimalmediums), um eine Osteoblastensuspension herzustellen; und
das Vermischen der erhaltenen Osteoblastensuspension mit einer Biomatrix, um ein Osteoblasten umfassendes therapeutisches Mittel herzustellen,
wobei die Biomatrix ein Gemisch aus Kollagen und Hydroxyapatit darstellt, und
wobei das Kollagen in einer Menge von 67 µg/ml bis 20 mg/ml zu der Osteoblastensuspension zugegeben wird, und das Hydroxyapatit in einer Menge von 30 µg/ml bis 3,4 mg/ml zu der Osteoblastensuspension zugesetzt wird.

2. Zusammensetzung zur Knochenbildung nach Anspruch 1, wobei der Schritt der Herstellung des therapeutischen Mittels weiterhin das Vermischen des Osteoblasten-Lösungsgemischs, worin die Biomatrix mit der Osteoblastensuspension vermischt wurde, mit einem Gerinnungsmittel umfasst.

3. Zusammensetzung zur Knochenbildung nach Anspruch 2, wobei der Mischschritt die folgenden Schritte umfasst:
das Vermischen des Osteoblasten-Lösungsgemischs mit 10 bis 100 IU/ml Thrombin als Gerinnungsmittel; und
das Vermischen des erhaltenen Lösungsgemischs, enthaltend das darin eingemischte Thrombin, mit 20 bis 100 mg/ml Fibrinogen als Gerinnungsmittel.

4. Zusammensetzung zur Knochenbildung nach Anspruch 1, wobei das Kollagen vor dem Vermischen mit der Osteoblastensuspension durch Zugabe einer neutralisierenden Lösung auf einen neutralen pH-Wert neutralisiert wird.

5. Zusammensetzung zur Knochenbildung nach Anspruch 3, wobei:
gefriergetrocknetes Thrombin als Thrombin vor der Zugabe zu dem Gemisch aus den Osteoblasten und der Biomatrix in flüssigem alpha-MEM, ergänzt mit Phosphat (PO₄³⁻)-Ionen als einen Knochenmineral-Bestandteil, gelöst wird und in der zweifachen Menge an Thrombin, welche in dem Kulturmedium enthalten ist, das mit (PO₄³⁻)-Ionen ergänzt wurde, zugegeben wird; und
gefriergetrocknetes Fibrinogen als Fibrinogen vor der Zugabe zu dem erhaltenen Osteoblasten-Lösungsgemisch in flüssigem alpha-MEM, ergänzt mit Calcium (Ca²⁺)-Ionen als einen Knochenmineral-Bestandteil, gelöst wird und in der zweifachen Menge an Fibrinogen, welche in dem Kulturmedium enthalten ist, das mit Calcium (Ca²⁺)-Ionen ergänzt wurde, zugegeben wird.

## Revendications

1. Composition pour formation d'os, préparée selon un procédé de préparation de compositions conçues pour la formation d'os, dans lequel on utilise un mélange d'ostéoblastes et d'une bio-matrice et qui comporte les étapes suivantes :
- cultiver et faire proliférer des ostéoblastes et des cellules précurseurs d'ostéoblastes, isolés à partir d'un tissu osseux, dans du milieu MEMα (variante alpha de milieu essentiel minimal), afin de préparer une suspension d'ostéoblastes ;
- et mélanger la suspension d'ostéoblastes ainsi obtenue avec une bio-matrice, pour en faire un agent thérapeutique à base d'ostéoblastes ;
et dans lequel procédé la bio-matrice est un mélange de collagène et d'hydroxy-apatite, et l'on ajoute à la suspension d'ostéoblastes du collagène,
en une quantité de 67 µg/mL à 20 mg/mL, et de l'hydroxy-apatite, en une quantité de 30 µg/mL à 3,4 mg/mL.

2. Composition pour formation d'os, conforme à la revendication 1, pour laquelle l'étape de préparation de l'agent thérapeutique comporte en outre le fait de mélanger avec un coagulant la solution mêlée d'ostéoblastes que l'on a obtenue en mélangeant la bio-matrice avec la suspension d'ostéoblastes.

3. Composition pour formation d'os, conforme à la revendication 2, pour laquelle l'étape de mélange comporte :
- le fait de mélanger la solution mêlée d'ostéoblastes avec de la thrombine, utilisée à raison de 10 à 100 UI/mL en tant que coagulant,
- et le fait de mélanger la solution mêlée résultante, contenant de la thrombine mélangée dedans, avec du fibrinogène, utilisé à raison de 20 à 100 mg/mL en tant que coagulant.

4. Composition pour formation d'os, conforme à la revendication 1, pour laquelle le collagène, avant d'être mélangé à la suspension d'ostéoblastes, est neutralisé, amené à pH neutre, par addition d'une solution de neutralisation.

5. Composition pour formation d'os, conforme à la revendication 3, pour laquelle :
- avant d'être ajoutée au mélange d'ostéoblastes et de bio-matrice, de la thrombine lyophilisée, utilisée en tant que thrombine, est dissoute dans du milieu MEMα liquide auquel on avait ajouté des ions phosphates PO₄³⁻ en tant que composant minéral de l'os, et elle est ajoutée de manière à donner le double de la quantité de thrombine contenue dans le milieu de culture additionné d'ions phosphates PO₄³⁻ ;
- et avant d'être ajouté au mélange d'ostéoblastes et de bio-matrice, du fibrinogène lyophilisé, utilisé en tant que fibrinogène, est dissous dans du milieu MEMα liquide auquel on avait ajouté des ions de calcium Ca²⁺ en tant que composant minéral de l'os, et il est ajouté de manière à donner le double de la quantité de fibrinogène contenue dans le milieu de culture additionné d'ions de calcium Ca²⁺.
